# EUROPEAN PATENT SPECIFICATION

(11) **EP 2 063 869 B1**
(45) Date of publication and mention of the grant of the patent: **05.03.2014**
(21) Application number: 07837739.7
(22) Date of filing: 05.09.2007
(51) Int. Cl.: A61K 9/20, A61K 9/08

(54) **SOFT CHEWABLE VETERINARY ANTIBIOTIC FORMULATIONS**
WEICHE KAUBARE TIERÄRZTLICHE ANTIBIOTISCHE FORMULIERUNGEN
FORMULATIONS ANTIBIOTIQUES VÉTÉRINAIRES SOUS FORME MÂCHABLE TENDRE

(30) Priority: 07.09.2006 US 842877 P
(43) Date of publication of application: 03.06.2009
(73) Proprietor: Merial Ltd., Duluth, GA 30096 (US)
(72) Inventor: SOLL, Mark, David, Alpharetta, GA 30005 (US); BOECKH, Albert, Ringoes New jersey 08551-1010 (US); TEJWANI-MOTWANI, Monica, Somerset, NJ 08873 (US); WARANIS, Robert, P., Annandale, NJ 08801 (US)
(74) Representative: Harding, Charles Thomas
(86) International application number: PCT/US2007/019357
(87) International publication number: WO 2008/030469

(56) References cited:
- WO-A-03/053420
- WO-A-2004/016252

## Description

### FIELD OF THE INVENTION

This application relates to formulations for combating bacterial infections in animals. In particular, this invention provides for improved long-acting oral formulations for systemic delivery of antibiotics, which are designed to achieve high bioavailability.

### BACKGROUND OF THE INVENTION

Antibiotics are a class of drugs that destroy or inhibit the growth of certain types of bacteria, and are commonly used to effectively control a variety of acute and chronic bacterial infectious diseases in birds and animals. Antibiotic therapy may result in killing the microorganism (bactericidal drugs) or inhibiting bacterial growth (bacteriostatic drugs). Antibiotics are classified as broad-spectrum or narrow-spectrum, depending on the types of bacteria they can kill or inhibit. The broad-spectrum antibiotics have antimicrobial effect on both the Gram-positive and Gram-negative bacteria, whereas the narrow-spectrum antibiotics only affect either the Gram-positive or the Gram-negative bacterial strains. There are five major groups of antibiotics that are classified by primary mechanism of action: cell wall synthesis inhibitors, cell membrane inhibitors, protein synthesis inhibitors, nucleic acid effectors, and folate inhibitors.

The kind of antibiotic, the time period for treatment, and the route of administration all vary based on the disease conditions and animal species. Therefore, it is generally helpful to discuss animals that are treated with antibiotics as members of one of three major groups: companion animals, food animals including poultry, and utility animals such as horses, which may also be considered companion animals, depending upon their use.

Antibiotic therapy for food animals usually does not extend beyond 5-10 days, while treatment of companion animals may extend for weeks or months for many chronic conditions. For example, some of the pathological conditions in dogs and cats, including chronic skin diseases, chronic otitis, chronic dermatitis, urinary tract infections, penetrating wounds and post-surgical treatment, may require prolonged or repeated systemic antibiotic administration. Long-term antibiotic therapy is also often required in cases of bacterial osteomyelitis.

Generally, antibiotics are administered by a variety of routes including, for example, oral ingestion, topical application or parental administration. The particular route of administration selected by the practitioner depends upon factors such as the physiochemical properties of the pharmaceutical or therapeutic agent, the condition of the host, and economic factors.

For example, one method of formulating a therapeutic agent for oral, topical, dermal or subdermal administration is to formulate the therapeutic agent as a paste or as an injectable formulation and reference is made to U.S. application Ser. No. 09/504,741, filed Feb. 16, 2000, issued September 7, 2004 as U.S. Patent 6,787,342, entitled PASTE FORMULATIONS; Ser. No. 09/346,905, filed Jul. 2, 1999, issued May 29, 2001 as U.S. Patent 6,239,112, entitled WATER MISCIBLE MACROLIDE SOLUTIONS; Ser. No. 09/112,690, filed Jul. 9, 1999, issued September 28, 1999 as U.S. Patent No. 5,958,888, entitled WATER MISCIBLE MACROLIDE SOLUTIONS; Ser. No. 08/675,380, filed July 2, 1996, issued March 3, 1998 as U.S. Patent No. 5,723,447, entitled WATER MISCIBLE ERYTHROMYCIN SOLUTIONS; Ser. No. 09/152,775, filed Sep. 14, 1998, issued January 16, 2001 as U.S. Patent No. 6,174,540, entitled LONG ACTING INJECTIBLE FORMULATIONS CONTAINING HYDROGENATED CASTOR OIL; and Ser. No. 09/271,098, filed March 18, 1999, issued May 11, 2004 as U.S. Patent No. 6,733,767, entitled LIQUID POLYMERIC COMPOSITIONS FOR CONTROLLED RELEASE OF BIOACTIVE SUBSTANCES.

Other methods of formulating therapeutic agents include placing the therapeutic agent in a solid or liquid matrix for oral delivery. These methods include chewable drug-delivery formulations. One problem associated with oral formulations is that the therapeutic agent often provides an unpleasant taste, aroma, or mouth feel to the formulation, which cause, especially in the situation with animals, the oral formulation to be rejected by the patient. See, e.g., U.S. Pat. No. 5,380,535 to Geyer et al., which provides for a lipid based, chewable formulations for oral delivery of therapeutic agents, such as aspirin, ibuprofen or erythromycin, which are unpalatable to humans; U.S. Pat. No. 5,894,029 to Brown et al., which provides for dried puff pet foods comprising farinaceious materials, proteinaceous materials, such as meats or vegetable protein sources, and optionally medicaments or vitamins; or U.S. Pat. No. 5,637,313 to Chau et al., which describes chewable dosage forms comprising a water soluble matrix comprising hydrogenated starch hydrolystate bulking agent and a water insoluble bulking agent. Reference is also made to Ser. No. 10/745,784, filed December 23, 2003, now pending, entitled NON-ANIMAL PRODUCT CONTAINING VETERINARY FORMULATIONS; and Ser. No. 10/222,559, filed August 16, 2002, now pending, entitled NON-ANIMAL PRODUCT CONTAINING VETERINARY FORMULATIONS.

Traditionally, in veterinary formulations, palatability had been achieved by the inclusion of animal byproducts or flavors derived from animal sources into the formulation. For example, it is customary to include attracts, such as chicken powder, liver powder, beef, ham, fish, or rawhide-derived products in dog chews to make the chew palatable to the dog. See, e.g., U.S. Patent 6,086,940; U.S. Patent 6,093,441; U.S. Patent 6,159,516; U.S. Patent 6,110,521; U.S. Patent 5,827,565; U.S. Patent 6,093,427, all to Axelrod et al. However, the use of animal products or byproducts or flavors derived from animal sources have recently fallen into disfavor because of the possibility of chemical or biological contamination, which lead to toxicity or diseases such as bovine spongiform encephalopathy. Hence, there is a need for oral veterinary formulations that do not contain animal products, byproducts, or flavors derived from animal sources while still exhibiting good organoleptic properties. While non-animal derived products such as valerian plants are know as scent attractants in food products or pet toys (U.S. Patent 5,785,382 to Childers-Zadah) or animal chews that contain fruit flavors as the attractant (see, U.S. Patents 6,274,182; 6,200,616 and 6,126,978 to Axelrod et al.), these patents do not describe using valerian plants or fruit flavors in oral formulations in which the pharmaceutical agents needs to be masked.

Another problem associated with oral formulations relates to "bioavailability", which indicates the percentage of a drug dose which reaches its site of action, or a biological fluid, from which the drug has access, to its site of action (Grant R. Wilkinson, Goodman & Gilman's The Pharmacological Basis of Therapeutics, Tenth Ed., 5 (Hardman, J.G., Limbird, L.E., and Gilman, A.G., eds., McGraw-Hill, 2001) (1941). The bioavailability of drugs is a complex issue. For example, a drug given orally must be absorbed first from the stomach and intestine, but this may be limited by the characteristics of the dosage form and/or the drug's physicochemical properties. In addition drug then passes through the liver, where metabolism and/or biliary excretion may occur before it reaches the systemic circulation. Accordingly, a fraction of the administered and absorbed dose of drug will be inactivated or diverted before it can reach the general circulation and be distributed to its sites of action. If the metabolic or excretory capacity of the liver for the agent in question is large, bioavailability will be substantially reduced (the so-called first pass effect). This decrease in availability is a function of the anatomical site from which absorption takes place; other anatomical, physiological, and pathological factors can influence bioavailability and the choice of the route of administration must be based on an understanding of these conditions (Grant R. Wilkinson, Goodman & Gilman's The Pharmacological Basis of Therapeutics, Tenth Ed., 5 (Hardman, J.G., Limbird, L.E., and Gilman, A.G., eds., McGraw-Hill, 2001) (1941).

One obvious way to change the bioavailability of a therapeutic agent is to change the route of administration from, for example, oral to parenteral. However, the use of parenteral injection may not always be appropriate. For example, intravenous injection has an increased risk of adverse effects and is not suitable for oily solutions or insoluble substances. Subcutaneous injections are not suitable for large volumes and may present possible pain or necrosis from irritating substances. Other strategies include increasing drug potency, changing dosage regimens, or using combination therapies. Furthermore, the choice of pharmaceutical formulation plays a role in rendering the therapeutic agent effective upon administration.

Antibiotic usage in veterinary medicine presents other unique considerations. Animal patients vary from small companion animals and birds that live in intimate proximity to their owners to pastured food and fiber producing animals with little human contact. The animal species, their human contact, temperament, size, use, emotional and economic value, and pathological conditions are all important factors that must be considered in selecting an appropriate type of antibiotic and administration route for therapy.

The particular dosage form varies based upon the kind of antibiotic used, the animal species being treated, and on whether the type of infection being treated requires local or systemic delivery. The advantage of local antibiotic therapy compared with systemic therapy is that a high concentration of antibacterial is delivered locally, thus avoiding the adverse effects that are associated with systemic antibacterial therapy. In general, companion and utility animals may be treated with a greater variety of therapeutic options than food animals, which are generally treated through systemic antibiotic administration. Local delivery of antibiotic is preferred or practical for some types of diseases in companion and utility animals. For example, doxycycline-loaded biodegradable polymer gel has been used to treat periodontal disease in beagles. On the other hand in horses, antibiotics are commonly used systemically for treatment of respiratory disease, wound infections, sinus infections, and neonatal sepsis. Because of the large size of the horses and susceptibility to antibiotic induced diarrhea and colitis, there exists a need to improve localized delivery of antibiotics in the equine patients.

The common approaches for systemic delivery of antibiotics are through oral and parenteral administration. The routes of parenteral injection could be intravenous, intramuscular or subcutaneous. However, intravenous administration may not be feasible or practical in species other than companion animals and utility animals such as horses, due to labor cost and management practices.

Antibiotics are used for three major purposes in farm animals: (a) to treat an individual or an outbreak of bacterial infection (treatment), (b) to prevent outbreaks of bacterial disease in animals at risk during certain phases of production (prophylaxis) and (c) to use the antibiotics in animal feed for growth promotion effects (growth promoter). Growth promoters and some prophylactic antibiotics are normally administered orally via feed or drinking water.

Drinking water and feed medication are preferred for poultry, mainly because of the large number of birds involved. However, therapeutic levels of antibiotics may not be achieved due to inadequate feed or water uptake by an individual sick bird, instability of the antibiotics in feed or water, or inappropriate feeding time and techniques. Therefore, in the case of serious disease, parenteral administration of the antibiotics for the sick birds can be a viable alternative; however, the therapy is rarely used. Parenteral administration of the antibiotics is time and labor consuming for the owner, and stressful for the sick birds, because multiple injections of a conventional injectable formulation are often required.

Parenteral administration of antibiotics is often preferred as a treatment mode for food animals. Therefore, antibiotic treatment of pastured animals or large companion animals generally requires confinement of these animals for the duration of therapy. However, repeated restraint and administration within a relatively short period of time add to the stress of illness and may complicate convalescence and recovery. Even docile animals tend to become fractious and uncooperative after multiple days of parenteral therapy.

It is therefore evident from the foregoing description that there are advantages of systemic or local delivery of long-acting antibiotic formulations to food producing and companion animals, and birds for the treatment of infectious diseases. Some of these advantages include improved patient compliance, convenience for the owner and veterinarians, and improved cost effectiveness of treating bacterial diseases. Long-acting antibiotic formulations can even reduce the amount of antibiotics used for therapy and/or prophylaxis in food animals, since the convenient and easily administered long-acting formulations make it possible to treat each affected animal in a more efficient and effective manner.

Several different approaches to develop long-acting antibiotic formulations have been explored. These include formulating oral dosage forms, injectable formulations such as suspensions, concentrated solutions, injectable gels and microparticles and implants. The selection of the development approach of long-acting antibiotic formulations is determined by the intended application criteria, such as type of disease, systemic or local therapy, short-term or long-term therapy and type of animals being treated.

Biodegradable polymers have been used in parenteral controlled release formulations of bioactive compounds. Gels prepared with biodegradable polymers such as poly(lactide-co-glycolide), poly(lactic acid) and polyoxyethylene polyoxypropylene block copolymers (poloxamers or, LUTROL® F) and biocompatible, non-toxic solvents, such as triethyl citrate and acetyl triethyl citrate or water have been used to develop long-acting antibiotics formulations. The reversible thermal gelation characteristics of the formulations allowed the liquid injection to gel at the injection site at body temperature.

In one approach the polymer is fabricated into microspheres that may be injected via syringe, and the bioative compound is entrapped within the microspheres. This approach has not proved to be practical in part due to the difficulty in the manufacturing procedure for producing sterile and reproducible products, and the high cost of manufacturing. In another approach the biodegradable polymer and the bioactive material are dissolved in a biocompatible water-miscible solvent to provide a liquid composition. When the liquid composition is injected into the body, the solvent dissipates into the surrounding aqueous environment, and the polymer forms a solid depot from which the bioactive material is released.

European Patent Application 0537559 concerns polymeric compositions having a thermoplastic polymer, rate modifying agent, water soluble bioactive material and water-miscible organic solvent. Upon exposure to an aqueous environment (e.g. body fluids) the liquid composition is capable of forming a biodegradable microporous, solid polymer matrix for controlled release of water soluble or dispersible bioactive materials over about four weeks. The thermoplastic polymer may be, among many listed, polylactide, polyglycolide, polycaprolactone or copolymers thereof, and is used in high concentration (45 to 50%). The rate modifying agent may be, among many others listed, glycerol triacetate (triacetin); however, only ethyl heptanoate is exemplified; and the amount of the rate modifying agent is no more than 15%.

Indeed, with respect to the patent literature, reference is made to: U.S. PAT. NO. INVENTOR 4,150,108 Graham 4,329,332 Couvreur et al. 4,331,652 Ludwig et al. 4,333,919 Kleber et al. 4,389,330 Tice et al. 4,489,055 Couvreur et al. 4,526,938 Churchill et al. 4,530,840 Tice et al. 4,542,025 Tice et al. 4,563,489 Urist 4,675,189 Kent et al. 4,677,191 Tanaka et al. 4,683,288 Tanaka et al. 4,758,435 Schaaf 4,857,335 Bohm 4,931,287 Bae et al. 5,178,872 Ohtsubo et al. 5,252,701 Jarrett et al. 5,275,820 Chang 5,478,564 Wantier et al. 5,540,912 Roorda et al. 5,447,725 Damani et al. 5,599,852 Scopelianos et al. 5,607,686 Totakura et al. 5,609,886 Wantier et al. 5,631,015 Bezwada et al. 5,654,010 Herbert et al. 5,700,485 Johnson et al. 5,702,717 Berde et al. 5,711,968 Tracy et al. 5,733,566 Lewis 4,938,763 Dunn et al. 5,077,049 Dunn et al. 5,278,201 Dunn et al. 5,278,202 Dunn et al. 5,288,496 Lewis 5,324,519 Dunn et al. 5,324,520 Dunn et al. 5,340,849 Dunn et al. 5,368,859 Dunn et al. 5,401,507 Lewis 5,419,910 Lewis 5,427,796 Lewis 5,487,897 Polson et al. 5,599,552 Dunn et al. 5,632,727 Tipton et al. 5,643,595 Lewis 5,660,849 Polson et al. 5,686,092 Lewis et al. 5,702,716 Dunn et al. 5,707,647 Dunn et al. 5,717,030 Dunn et al. 5,725,491 Tipton et al. 5,733,950 Dunn et al. 5,736,152 Dunn et al. 5,744,153 Yewey et al. 5,759,563 Yewey et al. 5,780,044 Yewey et al.

These documents tend to provide compositions that form a solid, gel or coagulated mass; for instance, a significant amount of polymer is contemplated in these documents, akin to European Patent Application 0537559.

Mention is also made of: Shah et al (J. Controlled Release, 1993, 27:139-147), as relating to formulations for sustained release of bioactive compounds containing various concentrations of poly(lactic-co-glycolic) acid copolymer (PLGA) dissolved in vehicles such as triacetin; Lambert and Peck (J. Controlled Release, 1995, 33:189-195), as a study of the release of protein from a 20% PLGA solution in N-methylpyrrolidone exposed to aqueous fluid; and Shivley et al (J. Controlled Release, 1995, 33:237-243), as a study of the solubility parameter of poly(lactide-co-glycolide) copolymer in a variety of solvents, and the in vivo release of naltrexone from two injectable implants (5% naltrexone in either 57% PLGA and 38% N-methylpyrrolidone or 35% PLGA and 60% N-methylpyrrolidone).

Various other gel-forming agents have been studied for usefulness as carriers for therapeutic agents, for example poloxamers. Poloxamers are a family of more than 30 different nontoxic nonionic surface active agents. Concentrated aqueous solutions of many of the poloxamers form gels, a property that reverses upon a decrease in temperature, whereupon the gel reverts to a liquid.

The use of poloxamers as delivery vehicles, such as controlled or sustained release systems, gels, microemulsionsand nanoparticles may provide enhanced solubility of therapeutic agents, enhanced bioavailability, lengthened contact at specifically selected sites in the body, combined with the reduction in quantity of applied drug. All of these aspects may be exploited in order to optimize systemic and minimize side effects of active drugs.

Chowdhury et al., U.S. Publication No. 20040087520, in part discusses the usefulness of poloxamers for *topical or ophthalmic delivery* of various therapeutic agents, including antibiotics. In this regard, this reference is primarily involved with studies of localized application of antibiotics at a surgical site to prevent surgical site infections.

Poloxamers have also been investigated for usefulness in controlled release injectable gel formulations. For example, when injected intramuscularly, the formulation forms a depot for the controlled release of drug by gelling at body temperature. Paavola, et al., investigated a method of delaying the action of a local anesthetic, lidocaine, in post-operative and chronic pain using a low-viscosity gel containing a poloxamer (Paavola, A. et al., Pharm. Res. Vol. 12, No. 12, 1995). The 2% lidocaine-containing gels were evaluated in rats. Based on the results, compared to other carriers, the poloxamer gel was held to be the most effective, providing release of lidocaine for up to 240 minutes. The reference did not discuss the feasibility of using gels as injectable sustained-release vehicles for antibiotics or any other therapeutic agents.

Another formulation approach of developing long acting injectable formulations is to prepare concentrated solutions of antibiotics for injection, using suitable pharmaceutically acceptable water-miscible solvents such as polyethylene glycol, propylene glycol, n-methyl pyrrolidone, and 2-pyrrolidone. After an intramuscular or subcutaneous injection of the concentrated antibiotic solution, the drug precipitates at the injection site since the water-miscible solvent is carried away or diluted by the biological fluids, or absorbed rapidly from the injection site. The precipitated drug particles are slowly dissolved in the biological fluid at the site of injection, and the dissolved drug is absorbed into the blood stream.

WO 2004/016252 A discloses a chewable veterinary formulation. Soy protein fines may be used as a filler.

Although most of the antibiotics currently on the market can generally be used in any animal species, developing a long-acting formulation which is suitable requires consideration of the size of animal species, physiological features of the animal, diseases to be treated, and the economic and emotional interest of the animal owners.

With all of the above factors at play in the development of antibiotic formulations, it remains a challenge to develop long-acting injectable formulations that remain effective for a sufficiently long time in order that a single injection is all that is necessary. The present invention fulfills this long-felt need.

### SUMMARY OF THE INVENTION

The present invention relates to novel chewable veterinary formulations that provide bioavailability of antibiotics that is comparable to a conventional capsule product. This invention further provides for improved veterinary formulations or which possess good consistency and acceptability by the animal, as well as a process to prepare said veterinary formulations.

The present invention encompasses a chewable veterinary formulation which comprises an antibiotic between 1 and 5% of the formulation, a hydrophobic material between 2 and 15%, soy protein fines between 20-60%, flavor between 5-30%, preservative between 0.2 to 1%, disintegrant between 2 and 10%, and solvent between 2 and 20%, wherein the antibiotic is clindamycin or a pharmaceutically acceptable salt or hydrate thereof. Most advantageously, the antibiotic is clindamycin HCl.

Advantageously, the chewable formulation comprises a hydrophobic material selected from the group consisting of glyceryl behenate, hydrogenated vegetable oil, stearic acid, glyceryl monostearate, glycerylpalmito stearate or cetyl alcohol. Most advantageously, the hydrophobic material is hydrogenated vegetable oil.

Advantageously, the chewable formulation comprises a flavor wherein the flavor is a hickory smoke flavor or a beef flavor.

Advantageously, the chewable formulation comprises a preservative selected from the group consisting of parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, propyl paraben, myristyl gama-picolinium chloride, paraben methyl, paraben propyl and quaternary ammonium compounds. More advantageously, the preservative is methylparaben and/or propylparaben.

Advantageously, the chewable formulation comprises a disintegrant selected from the group consisting of sodium starch glycolate, crospovidone, croscarmellose sodium, starch, micocrystalline cellulose, alginic acid, veegum, crospovidone, bentonite, and pregelatinized starch. More advantageously, the disintegrant is crospovidone.

Advantageously, the chewable formulation comprises a humectant is selected from the group consisting of propylene glycol, glycerin, polyethylene glycol 400 and polyethylene glycol 3350. More advantageously, the humectant is propylene glycol or purified water.

The chewable formulations are prepared according to methods conventional in the art, such as wet and dry granulation processes.

Advantageously, the process for preparing a chewable formulation comprises the steps of:
(a) blending the pharmaceutical agent, hydrophobic material, disintegrant, flavor;
(b) adding the water, preservative, and the humectant to the mixture from step (a) and mixing the mixture; and
(c) without drying, extruding the mixture.

Advantageously, administration of the chewable formulation of the present invention achieves bioavailability in an animal of a therapeutic agent that is comparable to commercially available products, and effectively treats bacterial infections in an animal.

The time course of treatment to be administered is easily determined by one skilled in the art. Advantageously, the animal receives treatment on days 0, 7, 14, 21, and 28.

These and other embodiments are disclosed or are obvious from and encompassed by, the following Detailed Description.

### BRIEF DESCRIPTION OF THE DRAWINGS

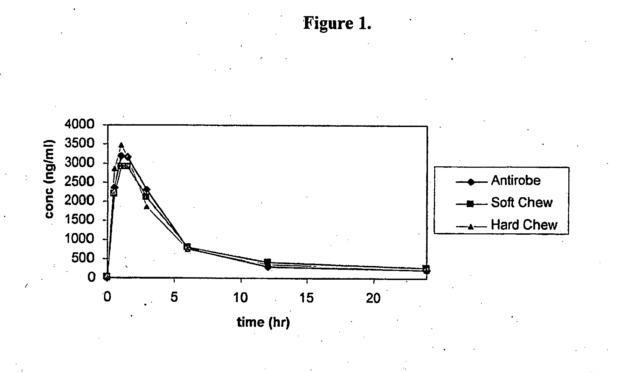

Figure 1 illustrates the mean concentrations of clindamycin in dog serum after treatment with soft chewable or hard chewable formulations as compared to the commercial product, ANTIROBE®.

### DETAILED DESCRIPTION

As used herein, the following terms have the meanings ascribed to them unless specified otherwise. In this disclosure, "comprises," "comprising," "containing" and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

The phrases "oral bioavailability" and "bioavailability upon oral administration" as used herein refer to the systemic availability (i.e., blood/plasma levels) of a given amount of antibiotic administered to a patient.

The term "clearance" as used herein refers to the removal of a substance from the blood, e.g., by renal excretion, expressed in terms of the volume flow of blood or plasma that would contain the amount of substance removed per unit time.

The term "half-life" as used herein refers to the period of time required for one-half of an amount of a substance to be lost through biological processes.

The term "bioavailability" as used herein refers to the physiological availability of a given amount of a drug, as distinct from its chemical potency. The term may also refer to the proportion of the administered dose which is absorbed into the bloodstream.

The term "animal" is used herein to include all vertebrate animals, including humans. It also includes an individual animal in all stages of development, including embryonic and fetal stages. As used herein, the term "production animals" is used interchangeably with "livestock animals" and refers generally to animals raised primarily for food. For example, such animals include, but are not limited to, cattle (bovine), sheep (ovine), pigs (porcine or swine), poultry (avian), and the like. As used herein, the term "cow" or "cattle" is used generally to refer to an animal of bovine origin of any age. Interchangeable terms include "bovine", "calf", "steer", "bull", "heifer", "cow" and the like. As used herein, the term "pig" is used generally to refer to an animal of porcine origin of any age. Interchangeable terms include "piglet", "sow" and the like.

The amount of therapeutic agent depends on the individual therapeutic agent, the animal being treated, the disease state, and the severity of the disease state. The determination of those factors is well within the skill level of the practitioner.

Preferred formulations are those containing about 2.5 to about 5% w/w of therapeutic agent.

Advantageously, the soft chewable formulation contains as an antibiotic, clindamycin, or salts thereof. Most preferred is clindamycin HCl in a range of 1-5% w/w.

For soft chewable formulations, the hydrophobic material may include surfactants of different degrees of hydrophobicity or hydrophilicity which can be prepared by reaction of alcohols or polyalcohols with a variety of natural and/or hydrogenated oils. Most commonly, the oils used are castor oil or hydrogenated castor oil, or an edible vegetable oil such as corn oil, olive oil, peanut oil, palm kernel oil, apricot kernel oil, soybean oil, or almond oil. Preferred alcohols include glycerol, propylene glycol, ethylene glycol, polyethylene glycol, sorbitol, and pentaerythritol. Among these alcohol-oil transesterified surfactants, preferred hydrophilic surfactants are PEG-35 castor oil (Incrocas-35), PEG-40 hydrogenated castor oil (Cremophor RH 40), PEG-25 trioleate (TAGAT® TO), PEG-60 corn glycerides (Crovol M70), PEG-60 almond oil (Crovol A70), PEG-40 palm kernel oil (Crovol PK70), PEG-50 castor oil (Emalex C-50), PEG-50 hydrogenated castor oil (Emalex HC-50), PEG-8 caprylic/capric glycerides (Labrasol), and PEG-6 caprylic/capric glycerides (Softigen 767). Preferred hydrophobic surfactants in this class include PEG-5 hydrogenated castor oil, PEG-7 hydrogenated castor oil, PEG-9 hydrogenated castor oil, PEG-6 corn oil (Labrafil® M 2125 CS), PEG-6 almond oil (Labrafil® M 1966 CS), PEG-6 apricot kernel oil (Labrafil® M 1944 CS), PEG-6 olive oil (Labrafil® M 1980 CS), PEG-6 peanut oil (Labrafil® M 1969 CS), PEG-6 hydrogenated palm kernel oil (Labrafil® M 2130 BS), PEG-6 palm kernel oil (Labrafil® M 2130 CS), PEG-6 triolein (Labrafil® M 2735 CS), PEG-8 corn oil (Labrafil® WL 2609 BS), PEG-20 corn glycerides (Crovol M40), and PEG-20 almond glycerides (Crovol A40).

Advantageously, the soft chewable formulation contains as a hydrophobic material, hydrogenated vegetable oil. Advantageously, the hydrogenated vegetable oil is present in the amounts of about 2-15% based upon total weight of formulation.

For soft chewable formulations, all fillers (or diluents) known in the soft chewable formulation art are contemplated. Non-limiting examples of fillers include soy protein, corn cob, or corn glutton meal.

Preferred formulations are those containing about 5 to 80% w/w of filler and especially preferred formulations are those containing about 10 to 70% w/w of filler.

The soft chewable formulation contains as a filler, soy protein fines, in amounts of about 20% to 60% based upon total weight of formulation.

For soft chewable formulations, flavors include those known in pet foods which are artificial and include, for example:

| | |
|---|---|
| DRY GARLIC-ADE OS | Formulated to provide a pungent garlic aroma. |
| LIQUID GARLIC-ADE OS | Same as dry garlic-ade in an oil miscible liquid form. |
| LIQUID GARLIC-ADE CONCENTRATE OM | Same as Dry Garlic-Ade but in a concentrated, oil miscible liquid form. |
| DRY ONION-ADE | Formulated to deliver an aroma and taste of cooked onions. |
| DRY GARLIC ONION-ADE | A dry blend of Garlic-Ade and Onion-Ade. |
| DRY CHEESE-ADE | A strong cheddar cheese flavor and aroma. |
| LIQUID CHEESE-ADE OM | An oil miscible, liquid version of Dry Cheese-Ade. |
| DRY CHICKEN-ADE | Formulated to provide the aroma of baked chicken. |
| LIQUID CHICKEN-ADE OS | An oil soluble liquid version of Dry Chicken-Ade. |
| LIQUID CHICKEN-ADE OS CONCENTRATE FFA | A concentrated form of Liquid Chicken-Ade OS. |
| DRY LIVER-ADE | Formulated to provide the aroma and flavor of cooked liver. |
| LIQUID LIVER-ADE CONCENTRATE | A concentrated liquid version of Dry Liver-Ade. |
| DRY PET-ADE BEEF STEW | A blend of many flavor components which provide of beef stew. |
| LIQUID PET-ADE BEEF STEW OS | An oil soluble, liquid version of Dry Pet-Ade Beef Stew. |
| PET-ADE BEEF STEW CONCENTRATE | A concentrated liquid version of Dry Pet-Ade Beef Stew. |
| DRY BEEF-ADE | A dry flavor formulated to provide the appeal of a baking roast. |
| DRY FISH MEAL FLAVOR CONCENTRATE | A dry flavor formulated to provide the odor of fish meal. |
| LIQUID FISH MEAL FLAVOR CONCENTRATE | A liquid version of Dry Fish Meal Flavor. |
| DRY KANIN-KRAVE | A spicy bone marrow flavor. |
| DRY BACON-ADE | A dry flavor which provides the aroma of frying bacon. |

Sources for these flavors are well-know to a practitioner in this art. For example, Kermine Petfood Nutrisurance is a vegetarian flavor for pet food is sold by Kemine industries, Inc., Des Moines, IW. A discussion of commercial smoke flavorings is provided by Guillen et al. in J. Agr. and Food Chemistry vol. 4.

Preferred are the GRILLIN' line of grill flavors and blends marketed by the Red Arrow Products Company, LLC, Manitowoc, WI for human and pet food. These include GRILLIN' TYPE CB-200, GRILLIN' TYPE SD, GRILLIN' TYPE WS-50, GRILLIN' TYPE CN, GRILLIN'TYPE CB, GRILLIN' TYPE GS and GRILLIN' TYPE NBF.

Especially preferred are hickory smoked flavoring produced by combining torula yeast and an aqueous hickory smoke solution, sold by Red Arrow Products Co. as CHARTOR HICKORY or a hickory smoke flavoring produced by combining maltodextin with an aqueous hickory smoke solution, sold by Red Arrow Products Co. as CHARDEX HICKORY. Other flavors contemplated by the invention include those which impart a natural dry smoke flavor. These include CHARZYME (a smoke flavor produced by combining barley malt flour with an aqueous smoke flavor), CHARMAIZE (a smoke flavor produced by combining yellow flower and an aqueous smoke flavor) and CHARSALT (a blend of dendritic salt, aqueous smoke flavor, and hydrated silicon dioxide. All of these flavors may be obtained by Red Arrow Products Co. The determination of the amounts of flavor for a particular product is easily determined by a practitioner of this art.

Advantageously, the soft chewable formulation contains those flavors which provide a savory flavor. These flavors are well known to a practitioner of this art. Typical ranges are from 5-30% w/w. Advantageously, the flavor is a hickory smoke flavor or a beef flavor.

For soft chewable formulations, all preservatives known in the soft chewable formulation art are contemplated. Non-limiting examples include the parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, propyl paraben, myristyl gama-picolinium chloride, paraben methyl, paraben propyl and quaternary ammonium compounds and the like.

Advantageously, the preservative is methylparaben and/or propylparaben. The preservative is used in the formulation in amounts ranging from about 0.2 to about 1.0% w/w For soft chewable formulations, all disintegrants known in the soft chewable formulation art are contemplated. Non-limiting examples include sodium starch glycolate, crospovidone, croscarmellose sodium, starch, micocrystalline cellulose, alginic acid, veegum, crospovidone, bentonite, and pregelatinized starch.

Advantageously, the disintegrant is crospovidone.

The disintegrant is used in the formulation in the amounts ranging from about 2-10% w/w.

For soft chewable formulations, all humectants known in the soft chewable formulation art are contemplated. Non-limiting examples include propylene glycol, glycerin, polyethylene glycol 400 and polyethylene glycol 3350.

Advantageously, the humectant is propylene glycol or purified water. Advantageously, these humectants are present in amounts of about 2% to 20% based upon total weight of formulation.

Optionally, the chewable veterinary formulations may also include lubricants, such as polyethylene glycols (PEG's or CARBOWAX), corn oil, mineral oil, hydrogenated vegetable oils (STEROTEX OR LUBRITAB), peanut oil, magnesium stearate, soybean oil and/or castor oil. The inclusion and identity of a lubricant is readily determined by a practitioner of this art are present in amounts, for example, of about 0.01 to about 20%, based upon total weight in the composition.

Absorbents may also be added to the chewable veterinary formulations. Such compounds are well known in the art to the practitioner as well as their use in pastes. These compounds effectively prevent or alleviate the phase separation of the product during storage. Preferred absorbents include magnesium carbonate, calcium carbonate, potassium bicarbonate, sodium bicarbonate, starch, cellulose and its derivatives, or mixtures of absorbents, with magnesium carbonate being especially preferred. The inclusion of these compounds is optional with amounts of 0% to about 30%, 0 to about 15% or about 1% to about 15% or about 1% to about 10%, based on total weight of the formulation being especially preferred.

Antioxidants such as an alpha tocopheral, ascorbic acid, ascrobyl palmitate, fumeric acid, malic acid, sodium ascorbate, sodium metabisulfate, n-propyl gallate, BHA (butylated hydroxy anisole), BHT (butylated hydroxy toluene) monothioglycerol and the like, may be added to the present formulation. The antioxidants are generally added to the formulation in amounts of from about 0.01 to about 2.0%, based upon total weight of the formulation, with about 0.1 to about 1.0% being especially preferred.

Granulating solvents are well known to those skilled in this art. Non-limiting examples of such solvents are water, aqueous sorbitol solution, etc. Other compounds which can act as solvents include polyethylene glycol 3350, glycerol caprylate/caprate and polyglycolized glycerides (GELUCIRE).

Compounds which stabilize the pH of the formulation (pH modifiers) are also contemplated. Again, such compounds are well known to a practitioner in the art as well as, how to use these compounds. Buffering systems include, for example, systems selected from the group consisting of acetic acid/acetate, malic acid/malate, citric acid/citrate, tataric acid/tartrate, lactic acid/lactate, phosphoric acid/phosphate, glycine/glycimate, tris, glutamic acid/glutamates and sodium carbonate. Preferred ranges for pH include from about 4 to about 6.5.

Other compounds contemplated by the inventive formulations include complexing agents, such as cyclodextrins, PVP, PEG, ethyl lactate and niacinamide. Amounts of such compounds to be included in the inventive formulation are well known to a practitioner of the art. Also contemplated are therapeutic agents to be in the form of emulsions, liposomes or micelles.

Flow aids or glidants are also well known in the art and include, for example, silicon dioxide (CARBOSIL) or silica gel (SYLOID), talc, starch, calcium, stearate, magnesium stearate, and aluminum magnesium silicate (NEUSILIN). Amounts of flow aids are readily determined by a practitioner in this art and include for using about 0.01 to about 25%, based upon weight of total composition. Non-limiting examples of lubricants for the tablets include magnesium and calcium stearate and stearic acid. Again, the various lubricants are well known to a practitioner of this art as well as the amounts of these compounds. Ranges include from about 0.01 to about 20% based upon the total weight of formulation.

Further, the present invention provides for a method for enhancing the palatability of an oral veterinary formulation, which comprises including in the formulation a flavor that is 'liked' by dogs.

This invention further provides for a process for preparing a chewable veterinary formulation, which comprises the steps of:
(a) blending the pharmaceutical agent, hydrophobic material, disintegrant, flavor;
(b) adding the water, preservative, and the humectant to the mixture from step (a) and mixing the mixture; and
(c) without drying, extruding the mixture.

Often it is beneficial to administer a formulation that contains a combination of two or more antibiotics, which possess different activity, in order to obtain a composition with a broad spectrum of activity. The inventive oral formulations herein described may be used to co-administer more than one antibiotic.

The inventive formulations herein described may be used to treat a number of disease states by administering to the host in need thereof an effective amount of the formulation containing the pharmaceutical agent. The determining of a treatment protocol of a specific indication would be well within the skill level of a practitioner in the pharmaceutical or veterinary arts. The hosts include all animals, e.g. cats, dogs, cattle, sheep, horses, and pigs.

The inventive formulations herein described may be administered to a warm-blooded animals, such as cattle, sheep, pigs, cats, dogs, horses, llamas, deer, rabbits, skunks, raccoons, camels and the like, or birds. The amount of pharmaceutical active agent depends on the individual therapeutic agent, the animal being treated, the disease state, and the severity of the disease state. The determination of those factors is well within the skill level of the practitioner.

### EXAMPLES

### Example 1: Tablet And Soft Chewable Formulations Demonstrate Comparable Bioavailability To A Conventional Capsule Product.

Six healthy Beagle or mongrel dogs, 6.3 to 15.0 months of age, weighing 7.8 to 10.0 kg were studied in this randomized, five-period crossover study. Dogs were randomly assigned to one of three treatment sequences by lottery. Within each sequence, dogs received one of three treatments on Days 0, 7, 14, 21 and 28. On each treatment day, dogs received either clindamycin capsules (Group 1), soft chewables (Group 2) or chewable tablets (Group 3). All treatments were administered orally at a dose rate of at least 10 mg/kg.

Blood samples were collected prior to each treatment and at 0.5, 1, 1.5, 3, 6, 12 and 24 hours after each treatment. Figure 1 provides plasma concentration levels (ng/ml) of clindamycin at each time point. The results indicate that the mean concentration-time profiles were parallel, with the mean Cmax slightly higher for the commercial product, ANTIROBE.

The pharmacokinetic parameters among the three treatment groups were similar with average terminal half lives of 5-7 hr and average times to maximum concentration of 1.1-1.6 hr. The relative bioavailabilities of the soft and hard chews are 87 and 110% w/v, respectively compared to ANTIROBE. Additionally, the pharmacokinetic parameters were broadly similar between groups fed 1.5-3 hour post dose versus 6 hours post dose for all formulations.

### Analysis of Plasma Concentration of Clindamycin

A bioanalytical method for the determination of clindamycin from canine serum samples was developed using Reversed-Phase HPLC with UV Detection. All serum samples were extracted using a liquid-liquid extraction procedure and injected on an HPLC with UV absorption at 210 nm. Sets of fortified control samples to assess method performance, along with an unfortified control sample were included to assess any inherent interference.

Pharmacokinetic analysis was performed using WinNonlin software, version 4.0 (Pharsight Corporation, Mountain View, CA, 2002). The area under the plasma concentration-time curve (AUC) was calculated using the linear/logarithmic trapezoidal , method from 0 to the last point at which drug concentration was quantified [AUC(0-tₗₐₛₜ)]. Clearance and volume of distribution values, not corrected for bioavailability, were also calculated for each animal. The terminal elimination half life was calculated via linear regression of the last two to four nonzero values. Cₘₐₓ and Tₘₐₓ for each animal were taken as the highest observed concentration and time to that observation.

**Table 1. Summary of pharmacokinetic parameters for a capsule (ANTIROBE), soft chewable, or hard chewable at 10 mg/kg (nominal) clindamycin to dogs**

| | Antirobe Avg±SD (n=10) | Soft chew Avg±SD (n=10) | Hard chew Avg±SD (n=10) |
|---|---|---|---|
| AUC(0-tₗₐₛₜ) (ng·hr/mL) | 17400±10400 | 18300±9410 | 17300±10300 |
| Cₘₐₓ (ng/mL) | 3720±1380 | 3070±981 | 3640±1060 |
| Tₘₐₓ (hr) | 1.25±0.72 | 1.55±0.80 | 1.10±0.32 |
| T_{½} (hr) | 5.02±2.26 | 5.75±4.65 | 6.97±6.82 |
| V/F (mL/kg) | 4700±2760 | 4340±3640 | 5800±5330 |
| Cl/F (mL/hr/kg) | 678±341 | 644±393 | 740±498 |

| | | | |
|---|---|---|---|
| Arithmetic averages; Values rounded to 3 significant digits; AUC = Area Under the Curve; Cₘₐₓ = Peak Concentration; Tₘₐₓ = Time to Peak Concentration; T_{½} = terminal elimination half life; V/F = apparent volume of distribution (not corrected for bioavailability); CL/F = clearance (not corrected for bioavailability) | | | |

### Example 2: Preferred Soft Chewable Formulation

Table 2 provides the preferred concentrations of active ingredient and excipients for soft chewable formulations.

**Table 2.**

| **#** | **Ingredient** | **%** |
|---|---|---|
| 1 | Clindamycin HCl | 1 - 5% |
| 2 | Hydrogenated vegetable Oil | 2 - 15% |
| 3 | Soy Protein Fines | 20 - 60% |
| 4 | Flavor | 5 - 30% |
| 5 | Preservative | 0.2 - 1.0% |
| 6 | Disintegrant | 2 - 10% |
| 7 | Propylene Glycol / Purified water / other ingredients | 2 - 20% |

Furthermore, the following is discribed (not part of the invention):
1. A chewable antibiotic formulation comprising an antibiotic, hydrophobic material, soy protein fines, a disintegrant, a solvent, and optionally a flavor, and optionally a preservative.
2. The formulation according to paragraph 1 wherein the antibiotic is between 1 and 5% of the formulation, the hydrophobic material is between 2 and 15%, the soy protein fines is between 20-60%, the flavor is between 5-30%, the preservative is between 0.2 to 1%, the disintegrant is between 2 and 10%, and the humectant is between 2 and 20%.
3. The formulation according to paragraph 2 wherein the antibiotic is selected from the group consisting of amikacin, aminosalicyclic acid, amoxicillin, amoxicillin and clavulanate potassium, ampicillin, azithromycin, bacampicillin, bacitracin, capreomycin, carbenicillin, carbenicillin indanyl sodium, cefaclor, cefadroxil, cefaloridine, cefamandole, cefazolin, cefazolin sodium, cefepime, cefinetazole, cefixime, cefmetazole, cefodizime, cefonicid, cefoperazone, ceforanide, cefotaxime, cefotetan, cefoxitin, cefoxitin sodium, cefpirome, cefpodoxime, cefpodoxime proxetil, cefquinome, ceftaxidime, ceftibuten, ceftiofur, ceftizoxime, ceftriaxone, cefuroxime, cephacelor, cephadrine, cephalexin, cephalothin, cephamandole, cephapirin, cepharadine, cephprozil, chloramphenicol, chlortetracycline, ciprofloxacin, clarithromycin, clindamycin HCl, clindamycin or salts thereof, clindamycin phosphate, clofazimine, cloxacillin, colistin, co-triamoxazole, cycloserine, dalfopristin, danofloxacin, demeclocycline, dicloxacillin, difloxacin, dihydro-streptomycin, dirithromycin, docycycline, efrotomycin, enoxacin, enrofloxacin, ertapenem, erythromycin and salts thereof, ethambutol HCl and other salts, ethionamide, florfenicol, flumequine, fosfomycin, fosfomycin, gamithromycin, gatifloxacin, gentamycin, imipenem, imipenem-cilastin, isoniazid, kanamycin, levofloxacin, lincomycin, linezolid, lomefloxacin, loracarbef mafenide, marbofloxacin, meropenem, methenamine, methicillin, metronidazole, mezlocillin, minocycline, moxifloxacin, nafcillin, nalidixic acid, neomycin, netilmicin, nitrofurantoin, norfloxacin, novobiocin, ofloxacin, orbifloxacin, ormetoprim, oxacillin, oxytetracycline, paromomycin, penicillin G, penicillin G aqueous, penicillin G benzatine, penicillin G procaine, penicillin V, penicillin V penicillin salts and complexes, pentamidine, piperacillin, piperacillin sodium, piperacillin-tazobactam, polymixin B, pyrazinamide, rifampin, roxithromycin, salts of carbenicillin, silver sulfadiazine, sparfloxacin, spectinomycin, spiramycin, streptomycin, streptozocin, sufadimethoxine-ormetoprim, sulfacetamide, sulfacytine, sulfadiazine, sulfadimethoxine, sulfadimethoxine-trimethoprim, sulfamerazine, sulfamethazine, sulfamethixole, sulfapyridine, sulfapyrizine, sulfasalazine, sulfinethoxazole, sulfisoxazole, tazobactam, teicoplanin,, tetracycline, tetracycline HCl, tiamulin, ticarcillin, ticarcillin and clavulanate potassium, tilmicosin, tobramycin, trimethoprim, trimetrexate and ketolides, troleanomycin, trovafloxacin, tulathromycin, tylosin, vancomycin and ketolides such as telithromycin and HMR 3004.
4. The formulation according to paragraph 2 wherein the antibiotic is clindamycin or a pharmaceutically acceptable salt or hydrate thereof.
5. The formulation according to paragraph 2 wherein the hydrophobic material is selected from the group consisting of glyceryl behenate, hydrogenated vegetable oil, stearic acid, glyceryl monostearate, glycerylpalmito stearate or cetyl alcohol.
6. The formulation according to paragraph 2 wherein the hydrophobic material is hydrogenated vegetable oil.
7. The formulation according to paragraph 2 where in the filler is selected from the group consisting of soy protein, corn cob, or corn glutton meal.
8. The formulation according to paragraph 2 where in the filler is soy protein.
9. The formulation according to paragraph 2 wherein the flavor is a hickory smoke flavor or a beef flavor.
10. The formulation according to paragraph 2 wherein the preservative is selected from the group consisting of parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, propyl paraben, myristyl gama-picolinium chloride, paraben methyl, paraben propyl and quaternary ammonium compounds.
11. The formulation according to paragraph 2 wherein the preservative is methylparaben and/or propylparaben.
12. The formulation according to paragraph 2 wherein the disintegrant is selected from the group consisting of sodium starch glycolate, crospovidone, croscarmellose sodium, starch, micocrystalline cellulose, alginic acid, veegum, crospovidone, bentonite, and pregelatinized starch.
13. The formulation according to paragraph 2 wherein the disintegrant is crospovidone.
14. The formulation according to paragraph 2 wherein the humectant is selected from the group consisting of propylene glycol, glycerin, polyethylene glycol 400 and polyethylene glycol 3350.
15. The formulation according to paragraph 2 wherein the humectant is propylene glycol or purified water.
16. A process for preparing a chewable veterinary formulation according to paragraph 1 which comprises the steps of:
   (a) blending the pharmaceutical agent, hydrophobic material, disintegrant, flavor;
   (b) adding the water, preservative, and the humectant to the mixture from step (a) and mixing the mixture; and
   (c) without drying, extruding the mixture.
17. A method of achieving bioavailability in an animal of a therapeutic agent that is comparable to commercially available products, comprising administering to an animal any one of the formulations of paragraphs 1 through 15.
18. A method for treating a bacterial infection in an animal comprising administering to the animal any one of the formulations of paragraphs 1 through 15.
19. The method of paragraph 18 wherein the animal receives treatment on days 0 ,7, 14, 21, and 28 comprising administering any of the formulations of paragraphs 1 through 15.
20. An antibiotic formulation comprising an antibiotic, a lactose carrier, mannitol, a binder and disintegrant, an aqueous solvent, and optionally a flavor, and optionally color.
21. The formulation according to paragraph 21 wherein the antibiotic is between 4 and 15%, the lactose carrier is between 40 and 80%, mannitol is between 5 and 15%, the binder and disintegrant are between 3 and 10%, the flavor is between 10 and 20%, the color is between 0.1 and 0.5%, and the aqueous solvent is of a concentration sufficient to q.s. to 100%.
22. The formulation according to paragraph 21 wherein the antibiotic is selected from the group consisting of amikacin, aminosalicyclic acid, amoxicillin, amoxicillin and clavulanate potassium, ampicillin, azithromycin, bacampicillin, bacitracin, capreomycin, carbenicillin, carbenicillin indanyl sodium, cefaclor, cefadroxil, cefaloridine, cefamandole, cefazolin, cefazolin sodium, cefepime, cefinetazole, cefixime, cefmetazole, cefodizime, cefonicid, cefoperazone, ceforanide, cefotaxime, cefotetan, cefoxitin, cefoxitin sodium, cefpirome, cefpodoxime, cefpodoxime proxetil, cefquinome, ceftaxidime, ceftibuten, ceftiofur, ceftizoxime, ceftriaxone, cefuroxime, cephacelor, cephadrine, cephalexin, cephalothin, cephamandole, cephapirin, cepharadine, cephprozil, chloramphenicol, chlortetracycline, ciprofloxacin, clarithromycin, clindamycin HCl, clindamycin or salts thereof, clindamycin phosphate, clofazimine, cloxacillin, colistin, co-triamoxazole, cycloserine, dalfopristin, danofloxacin, demeclocycline, dicloxacillin, difloxacin, dihydro-streptomycin, dirithromycin, docycycline, efrotomycin, enoxacin, enrofloxacin, ertapenem, erythromycin and salts thereof, ethambutol HCl and other salts, ethionamide, florfenicol, flumequine, fosfomycin, fosfomycin, gamithromycin, gatifloxacin, gentamycin, imipenem, imipenem-cilastin, isoniazid, kanamycin, levofloxacin, lincomycin, linezolid, lomefloxacin, loracarbef mafenide, marbofloxacin, meropenem, methenamine, methicillin, metronidazole, mezlocillin, minocycline, moxifloxacin, nafcillin, nalidixic acid, neomycin, netilmicin, nitrofurantoin, norfloxacin, novobiocin, ofloxacin, orbifloxacin, ormetoprim, oxacillin, oxytetracycline, paromomycin, penicillin G, penicillin G aqueous, penicillin G benzatine, penicillin G procaine, penicillin V, penicillin V penicillin salts and complexes, pentamidine, piperacillin, piperacillin sodium, piperacillin-tazobactam, polymixin B, pyrazinamide, rifampin, roxithromycin, salts of carbenicillin, silver sulfadiazine, sparfloxacin, spectinomycin, spiramycin, streptomycin, streptozocin, sufadimethoxine-ormetoprim, sulfacetamide, sulfacytine, sulfadiazine, sulfadimethoxine, sulfadimethoxine-trimethoprim, sulfamerazine, sulfamethazine, sulfamethixole, sulfapyridine, sulfapyrizine, sulfasalazine, sulfinethoxazole, sulfisoxazole, tazobactam, teicoplanin, , tetracycline, tetracycline HCl, tiamulin, ticarcillin, ticarcillin and clavulanate potassium, tilmicosin, tobramycin, trimethoprim, trimetrexate and ketolides, troleanomycin, trovafloxacin, tulathromycin, tylosin, vancomycin and ketolides such as telithromycin and HMR 3004.
23. The formulation according to paragraph 21 wherein the antibiotic is clindamycin or a pharmaceutically acceptable salt or hydrate thereof.
24. The formulation according to paragraph 21 wherein the antibiotic is clindamycin HCl.
25. The formulation according to paragraph 21 wherein the filler is selected from the group consisting of anhydrous lactose, hydrated lactose, sprayed dried lactose, crystalline maltose and maltodextrins.
26. The formulation according to paragraph 21 wherein the filler is lactose.
27. The formulation according to paragraph 21 wherein the binder is selected from the group consisting of polyvinyl pyrrolidone, povidone, starch, pregelatinized starch, gelatin, methylcellulose, hydroxypropyl cellulose, carboxymethyl cellulose sodium, ethylcellulose, sodium alginate, tragacanth, and acacia.
28. The formulation according to paragraph 21 wherein the binder is polyvinyl pyrrolidone.
29. The formulation according to paragraph 21 wherein the disintegrant is selected from the group consisting of sodium starch glycolate, crospovid one, croscarmellose sodium, starch, micocrystalline cellulose, alginic acid, veegum, crospovidone, bentonite, and pregelatinized starch.
30. The formulation according to paragraph 21 wherein the disintegrant is crospovidone.
31. The formulation according to paragraph 21 wherein the flavor is a hickory smoke flavor or a beef flavor.
32. The formulation according to paragraph 21 wherein the colorant is selected from the group consisting of dyes, an aluminum lake, caramel, colorant based upon iron oxide or a mixture of any of the foregoing.
33. The formulation according to paragraph 21 wherein the colorant is selected from the group consisting of organic dyes and titanium dioxide.
34. A process for preparing the chewable veterinary formulation according to paragraph 20 which comprises mixing the ingredients intimately and pressing into single scored tablets.
35. A method of achieving bioavailability in an animal of a therapeutic agent that is comparable to commercially available products, comprising administering to an animal any one of the formulations of paragraphs 20 through 33.
36. A method for treating a bacterial infection in an animal comprising administering to the animal any one of the formulations of paragraphs 20 through 33.
37. The method of paragraph 36 wherein the animal receives treatment on days 0 ,7, 14, 21, and 28 comprising administering any of the formulations of paragraphs 20 through 33.
38. The formulation according to paragraph 19 wherein the antibiotic is selected from the group consisting of amikacin, aminosalicyclic acid, amoxicillin, amoxicillin and clavulanate potassium, ampicillin, azithromycin, bacampicillin, bacitracin, capreomycin, carbenicillin, carbenicillin indanyl sodium, cefaclor, cefadroxil, cefaloridine, cefamandole, cefazolin, cefazolin sodium, cefepime, cefinetazole, cefixime, cefmetazole, cefodizime, cefonicid, cefoperazone, ceforanide, cefotaxime, cefotetan, cefoxitin, cefoxitin sodium, cefpirome, cefpodoxime, cefpodoxime proxetil, cefquinome, ceftaxidime, ceftibuten, ceftiofur, ceftizoxime, ceftriaxone, cefuroxime, cephacelor, cephadrine, cephalexin, cephalothin, cephamandole, cephapirin, cepharadine, cephprozil, chloramphenicol, chlortetracycline, ciprofloxacin, clarithromycin, clindamycin HCl, clindamycin or salts thereof, clindamycin phosphate, clofazimine, cloxacillin, colistin, co-triamoxazole, cycloserine, dalfopristin, danofloxacin, demeclocycline, dicloxacillin, difloxacin, dihydro-streptomycin, dirithromycin, docycycline, efrotomycin, enoxacin, enrofloxacin, ertapenem, erythromycin and salts thereof, ethambutol HCl and other salts, ethionamide, florfenicol, flumequine, fosfomycin, fosfomycin, gamithromycin, gatifloxacin, gentamycin, imipenem, imipenem-cilastin, isoniazid, kanamycin, levofloxacin, lincomycin, linezolid, lomefloxacin, loracarbef mafenide, marbofloxacin, meropenem, methenamine, methicillin, metronidazole, mezlocillin, minocycline, moxifloxacin, nafcillin, nalidixic acid, neomycin, netilmicin, nitrofurantoin, norfloxacin, novobiocin, ofloxacin, orbifloxacin, ormetoprim, oxacillin, oxytetracycline, paromomycin, penicillin G, penicillin G aqueous, penicillin G benzatine, penicillin G procaine, penicillin V, penicillin V penicillin salts and complexes, pentamidine, piperacillin, piperacillin sodium, piperacillin-tazobactam, polymixin B, pyrazinamide, rifampin, roxithromycin, salts of carbenicillin, silver sulfadiazine, sparfloxacin, spectinomycin, spiramycin, streptomycin, streptozocin, sufadimethoxine-ormetoprim, sulfacetamide, sulfacytine, sulfadiazine, sulfadimethoxine, sulfadimethoxine-trimethoprim, sulfamerazine, sulfamethazine, sulfamethixole, sulfapyridine, sulfapyrizine, sulfasalazine, sulfinethoxazole, sulfisoxazole, tazobactam, teicoplanin,, tetracycline, tetracycline HCl, tiamulin, ticarcillin, ticarcillin and clavulanate potassium, tilmicosin, tobramycin, trimethoprim, trimetrexate and ketolides, troleanomycin, trovafloxacin, tulathromycin, tylosin, vancomycin and ketolides such as telithromycin and HMR 3004.
39. The method of paragraph 22 wherein the animal receives treatment on any of days 0 ,7, 14, 21, and 28 comprising administering any of the formulations of paragraphs 38 through 44, wherein the formulation provides sustained concentrations of therapeutic agents for 7-10 days.

## Claims

1. A chewable antibiotic veterinary formulation comprising:
an antibiotic between 1 and 5%, a hydrophobic material between 2 and 15%, soy protein fines between 20-60%, a flavor between 5-30%, a preservative between 0.2 to 1%, a disintegrant between 2 and 10%, and a humectant between 2 and 20% of the formulation; wherein the antibiotic is clindamycin or a pharmaceutically acceptable salt or hydrate thereof.

2. The formulation according to claim 1 wherein the hydrophobic material is selected from the group consisting of glyceryl behenate, hydrogenated vegetable oil, stearic acid, glyceryl monostearate, glycerylpalmito stearate or cetyl alcohol.

3. The formulation according to claim 2 wherein the hydrophobic material is hydrogenated vegetable oil.

4. The formulation according to claim 1, wherein the flavor is a hickory smoke flavor or a beef flavor.

5. The formulation according to claim 1, wherein the preservative is selected from the group consisting of parabens (methylparaben and/or propylparaben), benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, propyl paraben, myristyl gama-picolinium chloride, paraben methyl, paraben propyl and quaternary ammonium compounds.

6. The formulation according to claim 5 wherein the preservative is methylparaben and/or propylparaben.

7. The formulation according to claim 1 wherein the disintegrant is selected from the group consisting of sodium starch glycolate, crospovidone, croscarmellose sodium, starch, micocrystalline cellulose, alginic acid, veegum, crospovidone, bentonite, and pregelatinized starch.

8. The formulation according to claim 7 wherein the disintegrant is crospovidone.

9. The formulation according to claim 1 wherein the humectant is selected from the group consisting of propylene glycol, glycerin, polyethylene glycol 400 and polyethylene glycol 3350.

10. The formulation according to claim 1 wherein the humectant is propylene glycol or purified water.

11. A process for preparing a chewable veterinary formulation according to any one of claims 1 to 10 which comprises the steps of:
(a) blending the pharmaceutical agent, hydrophobic material, disintegrant, flavor;
(b) adding the water, preservative, and the humectant to the mixture from step (a) and mixing the mixture; and
(c) without drying, extruding the mixture.

12. A chewable antibiotic veterinary formulation according to claims 1 to 10 for use in treating a bacterial infection in an animal.

13. A chewable antibiotic veterinary formulation for use according to claim 12 wherein the animal receives treatment on days 0, 7, 14, 21, and 28.

## Patentansprüche

1. Kaubare veterinärmedizinische Antibiotikaformulierung umfassend:
ein Antibiotikum zwischen 1 und 5%, ein hydrophobes Material zwischen 2 und 15%, Sojaproteinstaub zwischen 20 - 60%, ein Geschmacksstoff zwischen 5 - 30%, ein Konservierungsmittel zwischen 0,2 und 1%, ein Sprengmittel zwischen 2 und 10% und ein Feuchthaltemittel zwischen 2 und 20% der Formulierung; wobei das Antibiotikum Clindamycin oder ein pharmazeutisch verträgliches Salz oder Hydrat davon ist.

2. Formulierung nach Anspruch 1, wobei das hydrophobe Material ausgewählt ist aus der Gruppe bestehend aus Glycerylbehenat, hydriertem Pflanzenöl, Stearinsäure, Glycerylmonostearat, Glycerylpalmitostearat oder Cetylalkohol.

3. Formulierung nach Anspruch 2, wobei das hydrophobe Material hydriertes Pflanzenöl ist.

4. Formulierung nach Anspruch 1, wobei der Geschmacksstoff ein Hickoryrauchgeschmacksstoff oder Rindgeschmacksstoff ist.

5. Formulierung nach Anspruch 1, wobei das Konservierungsmittel ausgewählt ist aus der Gruppe bestehend aus Parabenen (Methylparaben und/oder Propylparaben), Benzalkoniumchlorid, Benzethoniumchlorid, Benzoesäure, Benzylalkohol, Bronopol, Butylparaben, Cetrimid, Chlorhexidin, Chlorbutanol, Chlorcresol, Ethylparaben, Imidurea, Methylparaben, Phenol, Phenoxyethanol, Phenylethylalkohol, Phenylquecksilberacetat, Phenylquecksilberborat, Phenylquecksilbernitrat, Kaliumsorbat, Natriumbenzoat, Natriumpropionat, Sorbinsäure, Thimerosal, Propylparaben, Myristil-gamma-picoliniumchlorid, Parabenmethyl, Parabenpropyl und quaternäre Ammoniumverbindungen.

6. Formulierung nach Anspruch 5, wobei das Konservierungsmittel Methylparaben und/oder Propylparaben ist.

7. Formulierung nach Anspruch 1, wobei das Sprengmittel ausgewählt ist aus der Gruppe bestehend aus Natriumstärkeglycolat, Crospovidon, Croscarmellosenatrium, Stärke, mikrokristalliner Cellulose, Alginsäure, Veegum, Crospovidon, Bentonit und Quellstärke.

8. Formulierung nach Anspruch 7, wobei das Sprengmittel Crospovidon ist.

9. Formulierung nach Anspruch 1, wobei das Feuchthaltemittel ausgewählt ist aus der Gruppe bestehend aus Propylenglycol, Glycerin, Polyethylenglycol 400 und Polyethylenglycol 3350.

10. Formulierung nach Anspruch 1, wobei das Feuchthaltemittel Propylenglycol oder gereinigtes Wasser ist.

11. Verfahren zur Herstellung einer kaubaren veterinärmedizinischen Formulierung nach einem der Ansprüche 1 bis 10, das die Schritte umfasst:
(a) Mischen von pharmazeutischem Wirkstoff, hydrophobem Material, Sprengmittel, Geschmacksstoff;
(b) Hinzufügen von Wasser, Konservierungsmittel und Feuchthaltemittel zu dem Gemisch aus Schritt (a) und Mischen des Gemischs; und
(c) Strangpressen des Gemischs ohne Trocknung.

12. Kaubare veterinärmedizinische Antibiotikaformulierung nach den Ansprüchen 1 bis 10 zur Verwendung bei der Behandlung einer bakteriellen Infektion bei einem Tier.

13. Kaubare veterinärmedizinische Antibiotikaformulierung zur Verwendung nach Anspruch 12, wobei das Tier die Behandlung an den Tagen 0, 7, 14, 21 und 28 erhält.

## Revendications

1. Formulation antibiotique vétérinaire à mâcher comprenant :
un antibiotique entre 1 et 5 %, un matériau hydrophobe entre 2 et 15 %, des protéines de soja fines entre 20 et 60 %, un arôme entre 5 et 30 %, un conservateur entre 0,2 et 1 %, un agent de délitement entre 2 et 10 %, et un humectant entre 2 et 20 % de la formulation ;
dans laquelle l'antibiotique est la clindamycine ou un sel pharmaceutiquement acceptable ou un hydrate de celle-ci.

2. Formulation selon la revendication 1, dans laquelle le matériau hydrophobe est choisi dans le groupe consistant en le béhénate de glycéryle, l'huile végétale hydrogénée, l'acide stéarique, le monostéarate de glycéryle, le palmitostéarate de glycéryle ou l'alcool cétylique.

3. Formulation selon la revendication 2, dans laquelle le matériau hydrophobe est une huile végétale hydrogénée.

4. Formulation selon la revendication 1, dans laquelle l'arôme est un arôme fumé au feu de bois ou un arôme boeuf.

5. Formulation selon la revendication 1, dans laquelle le conservateur est choisi dans le groupe consistant en les parabènes (méthylparabène et/ou propylparabène), le chlorure de benzalkonium, le chlorure de benzéthonium, l'acide benzoïque, l'alcool benzylique, le bronopol, le butylparabène, le cétrimide, la chlorhexidine, le chlorobutanol, le chlorocrésol, le crésol, l'éthylparabène, l'imidurée, le méthylparabène, le phénol, le phénoxyéthanol, l'alcool phényléthylique, l'acétate phénylmercurique, le borate phénylmercurique, le nitrate phénylmercurique, le sorbate de potassium, le benzoate de sodium, le propionate de sodium, l'acide sorbique, le thimérosal, le propylparabène, le chlorure de myristyl-gamma-picolinium, le méthylparabène, le propylparabène, et des composés d'ammonium quaternaire.

6. Formulation selon la revendication 5, dans laquelle le conservateur est le méthylparabène et/ou le propylparabène

7. Formulation selon la revendication 1, dans laquelle l'agent de délitement est choisi dans le groupe consistant en le glycolate d'amidon sodique, la crospovidone, la croscarmellose sodique, l'amidon, la cellulose microcristalline, l'acide alginique, le veegum, la crospovidone, la bentonite et l'amidon prégélatinisé.

8. Formulation selon la revendication 7, dans laquelle l'agent de délitement est la crospovidone.

9. Formulation selon la revendication 1, dans laquelle l'humectant est choisi dans le groupe consistant en le propylène glycol, la glycérine, le polyéthylène glycol 400 et le polyéthylène glycol 3350.

10. Formulation selon la revendication 1, dans laquelle l'humectant est le propylène glycol ou l'eau purifiée.

11. Procédé de préparation d'une formulation vétérinaire à mâcher selon l'une quelconque des revendications 1 à 10, qui comprend les étapes suivantes :
(a) le mélange de l'agent pharmaceutique, du matériau hydrophobe, de l'agent de délitement, de l'arôme ;
(b) l'addition de l'eau, du conservateur, et de l'humectant au mélange issu de l'étape (a) et le mélange du mélange ; et
(c) sans séchage, l'extrusion du mélange.

12. Formulation antibiotique vétérinaire à mâcher selon les revendications 1 à 10, pour une utilisation dans le traitement d'une infection bactérienne chez un animal.

13. Formulation antibiotique vétérinaire à mâcher pour une utilisation selon la revendication 12, dans laquelle l'animal reçoit le traitement aux jours 0, 7, 14, 21 et 28.
